# EUROPÄISCHE PATENTSCHRIFT

(11) **EP 1 068 331 B1**
(45) Veröffentlichungstag und Bekanntmachung des Hinweises auf die Patenterteilung: **29.11.2006**
(21) Anmeldenummer: 99919176.0
(22) Anmeldetag: 01.04.1999
(51) Int. Cl.: C12N 15/49, C12N 15/54, C07K 14/16, C12N 9/12, A61K 39/21, C12N 15/63

(54) **ARZNEIMITTEL ZUR INDUKTION ZYTOTOXISCHER T-ZELLEN**
MEDICAMENTS FOR INDUCING CYTOTOXIC T-CELLS
MEDICAMENTS POUR L'INDUCTION DE CELLULES T CYTOTOXIQUES

(30) Priorität: 03.04.1998 DE 19814925
(43) Veröffentlichungstag der Anmeldung: 17.01.2001
(73) Patentinhaber: Harrer, Thomas, Dr., 91054 Erlangen (DE)
(72) Erfinder: HARRER, Thomas, D-91054 Erlangen (DE)
(74) Vertreter: Dossmann, Gérard
(86) Internationale Anmeldenummer: PCT/EP1999/002249
(87) Internationale Veröffentlichungsnummer: WO 1999/051750

(56) Entgegenhaltungen:
- WO-A-94/28871
- WO-A-97/14436
- TANIGAKI, NOBUYUKI ET AL: "The peptide binding specificity of HLA-B27 subtypes" IMMUNOGENETICS (1994), 40(3), 192-8 , XP002111887
- KOWALSKI, HEINRICH ET AL: "Patr-A and B, the orthologs of HLA-A and B, present hepatitis C virus epitopes to CD8+ cytotoxic T cells from two chronically infected chimpanzees" J. EXP. MED. (1996), 183(4), 1761-75 , XP002111888
- HARRIER, ELLEN ET AL: "Recognition of the highly conserved YMDD region in the human immunodeficiency virus type 1 reverse transcriptase by HLA-A2-restricted cytotoxic T lymphocytes from an asymptomatic long-term nonprogressor" J. INFECT. DIS. (1996), 173(2), 476-9 , XP002112244

### SEQUENZPROTOKOLL

<110> Glaxo Group Ltd.
   Harrer Dr., Thomas
<120> Arzneimittel zur Induktion zytotoxischer T-Zellen
<130> 77673dm3
<140>
   <141>
<150> DE 19814925.5
   <151> 1998-04-03
<160> 42
<170> PatentIn Vers. 2.0
<210> 1
   <211> 10
   <212> PRT
   <213> Künstliche Sequenz
<220>
   <223> Die Beschreibung von Künstliche Sequenz:Peptid
<400> 1
   Ile Val Ile Tyr Gln Tyr Val Asp Asp Leu
   1 5 10
<210> 2
   <211> 10
   <212> PRT
   <213> Künstliche Sequenz
<220>
   <223> Die Beschreibung von Künstliche Sequenz:Peptid
<400> 2
   Ile Val Ile Cys Gln Tyr Val Asp Asp Leu
   1 5 10
<210> 3
   <211> 10
   <212> PRT
   <213> Künstliche Sequenz
<220>
   <223> Die Beschreibung von Künstliche Sequenz:Peptid
<400> 3
   Ile Val Ile Tyr Gln Tyr Ile Asp Asp Leu
   1 5 10
<210> 4
   <211> 10
   <212> PRT
   <213> Künstliche Sequenz
<220>
   <223> Die Beschreibung von Künstliche Sequenz:Peptid
<400> 4
   Ile Val Ile Cys Gln Tyr Ile Asp Asp Leu
   1 5 10
<210> 5
   <211> 10
   <212> PRT
   <213> Künstliche Sequenz
<220>
   <223> Die Beschreibung von Künstliche Sequenz:Peptid
<400> 5
   Ile Thr Ile Tyr Gln Tyr Val Asp Asp Leu
   1 5 10
<210> 6
   <211> 10
   <212> PRT
   <213> Künstliche Sequenz
<220>
   <223> Die Beschreibung von Künstliche Sequenz:Peptid
<400> 6
   Ile Thr Ile Cys Gln Tyr Val Asp Asp Leu
   1 5 10
<210> 7
   <211> 10
   <212> PRT
   <213> Künstliche Sequenz
<220>
   <223> Die Beschreibung von Künstliche Sequenz:Peptid
<400> 7
   Ile Thr Ile Tyr Gln Tyr Ile Asp Asp Leu
   1 5 10
<210> 8
   <211> 10
   <212> PRT
   <213> Künstliche Sequenz
<220>
   <223> Die Beschreibung von Künstliche Sequenz:Peptid
<400> 8
   Ile Thr Ile Cys Gln Tyr Ile Asp Asp Leu
   1 5 10
<210> 9
   <211> 10
   <212> PRT
   <213> Künstliche Sequenz
<220>
   <223> Die Beschreibung von Künstliche Sequenz:Peptid
<400> 9
   Ile Ile Ile Tyr Gln Tyr Val Asp Asp Leu
   1 5 10
<210> 10
   <211> 10
   <212> PRT
   <213> Künstliche Sequenz
<220>
   <223> Die Beschreibung von Künstliche Sequenz:Peptid
<400> 10
   Ile Ile Ile Cys Gln Tyr Val Asp Asp Leu
   1 5 10
<210> 11
   <211> 10
   <212> PRT
   <213> Künstliche Sequenz
<220>
   <223> Die Beschreibung von Künstliche Sequenz:Peptid
<400> 11
   Ile Ile Ile Tyr Gln Tyr Ile Asp Asp Leu
   1 5 10
<210> 12
   <211> 10
   <212> PRT
   <213> Künstliche Sequenz
<220>
   <223> Die Beschreibung von Künstliche Sequenz:Peptid
<400> 12
   Ile Ile Ile Cys Gln Tyr Ile Asp Asp Leu
   1 5 10
<210> 13
   <211> 10
   <212> PRT
   <213> Künstliche Sequenz
<220>
   <223> Die Beschreibung von Künstliche Sequenz:Peptid
<400> 13
   Met Val Ile Tyr Gln Tyr Val Asp Asp Leu
   1 5 10
<210> 14
   <211> 10
   <212> PRT
   <213> Künstliche Sequenz
<220>
   <223> Die Beschreibung von Künstliche Sequenz:Peptid
<400> 14
   Met Val Ile Cys Gln Tyr Val Asp Asp Leu
   1 5 10
<210> 15
   <211> 10
   <212> PRT
   <213> Künstliche Sequenz
<220>
   <223> Die Beschreibung von Künstliche Sequenz:Peptid
<400> 15
   Met Val Ile Tyr Gln Tyr Ile Asp Asp Leu
   1 5 10
<210> 16
   <211> 10
   <212> PRT
   <213> Künstliche Sequenz
<220>
   <223> Die Beschreibung von Künstliche Sequenz:Peptid
<400> 16
   Met Val Ile Cys Gln Tyr Ile Asp Asp Leu
   1 5 10
<210> 17
   <211> 9
   <212> PRT
   <213> Künstliche Sequenz
<220>
   <223> Die Beschreibung von Künstliche Sequenz:Peptid
<400> 17
   Val Ile Tyr Gln Tyr Val Asp Asp Leu
   1 5
<210> 18
   <211> 9
   <212> PRT
   <213> Künstliche Sequenz
<220>
   <223> Die Beschreibung von Künstliche Sequenz:Peptid
<400> 18
   Val Ile Cys Gln Tyr Val Asp Asp Leu
   1 5
<210> 19
   <211> 9
   <212> PRT
   <213> Künstliche Sequenz
<220>
   <223> Die Beschreibung von Künstliche Sequenz:Peptid
<400> 19
   Val Ile Tyr Gln Tyr Ile Asp Asp Leu
   1 5
<210> 20
   <211> 9
   <212> PRT
   <213> Künstliche Sequenz
<220>
   <223> Die Beschreibung von Künstliche Sequenz:Peptid
<400> 20
   Val Ile Cys Gln Tyr Ile Asp Asp Leu
   1 5
<210> 21
   <211> 9
   <212> PRT
   <213> Künstliche Sequenz
<220>
   <223> Die Beschreibung von Künstliche Sequenz:Peptid
<400> 21
   Leu Ile Tyr Gln Tyr Val Asp Asp Leu
   1 5
<210> 22
   <211> 9
   <212> PRT
   <213> Künstliche Sequenz
<220>
   <223> Die Beschreibung von Künstliche Sequenz:Peptid
<400> 22
   Leu Ile Cys Gln Tyr Val Asp Asp Leu
   1 5
<210> 23
   <211> 9
   <212> PRT
   <213> Künstliche Sequenz
<220>
   <223> Die Beschreibung von Künstliche Sequenz:Peptid
<400> 23
   Leu Ile Tyr Gln Tyr Ile Asp Asp Leu
   1 5
<210> 24
   <211> 9
   <212> PRT
   <213> Künstliche Sequenz
<220>
   <223> Die Beschreibung von Künstliche Sequenz:Peptid
<400> 24
   Leu Ile Cys Gln Tyr Ile Asp Asp Leu
   1 5
<210> 25
   <211> 11
   <212> PRT
   <213> Künstliche Sequenz
<220>
   <223> Die Beschreibung von Künstliche Sequenz:Peptid
<400> 25
   Thr Ile Leu Gln Tyr Val Asp Asp Ile Leu Leu
   1 5 10
<210> 26
   <211> 11
   <212> PRT
   <213> Künstliche Sequenz
<220>
   <223> Die Beschreibung von Künstliche Sequenz:Peptid
<400> 26
   Thr Ile Leu Gln Tyr Ile Asp Asp Ile Leu Leu
   1 5 10
<210> 27
   <211> 9
   <212> PRT
   <213> Künstliche Sequenz
<220>
   <223> Die Beschreibung von Künstliche Sequenz:Peptid
<400> 27
   Ile Leu Gln Tyr Val Asp Asp Ile Leu
   1 5
<210> 28
   <211> 9
   <212> PRT
   <213> Künstliche Sequenz
<220>
   <223> Die Beschreibung von Künstliche Sequenz:Peptid
<400> 28
   Ile Leu Gln Tyr Ile Asp Asp Ile Leu
   1 5
<210> 29
   <211> 11
   <212> PRT
   <213> Künstliche Sequenz
<220>
   <223> Die Beschreibung von Künstliche Sequenz:Peptid
<400> 29
   Thr Ile Val Gln Tyr Val Asp Asp Ile Leu Leu
   1 5 10
<210> 30
   <211> 11
   <212> PRT
   <213> Künstliche Sequenz
<220>
   <223> Die Beschreibung von Künstliche Sequenz:Peptid
<400> 30
   Thr Ile Val Gln Tyr Ile Asp Asp Ile Leu Leu
   1 5 10
<210> 31
   <211> 9
   <212> PRT
   <213> Künstliche Sequenz
<220>
   <223> Die Beschreibung von Künstliche Sequenz:Peptid
<400> 31
   Ile Val Gln Tyr Ile Asp Asp Ile Leu
   1 5
<210> 32
   <211> 9
   <212> PRT
   <213> Künstliche Sequenz
<220>
   <223> Die Beschreibung von Künstliche Sequenz:Peptid
<400> 32
   Ile Val Gln Tyr Ile Asp Asp Ile Leu
   1 5
<210> 33
   <211> 10
   <212> PRT
   <213> Künstliche Sequenz
<220>
   <223> Die Beschreibung von Künstliche Sequenz:Peptid
<400> 33
   Ile Leu Val Gln Tyr Val Asp Asp Ile Leu
   1 5 10
<210> 34
   <211> 10
   <212> PRT
   <213> Künstliche Sequenz
<220>
   <223> Die Beschreibung von Künstliche Sequenz:Peptid
<400> 34
   Ile Leu Val Gln Tyr Ile Asp Asp Ile Leu
   1 5 10
<210> 35
   <211> 10
   <212> PRT
   <213> Künstliche Sequenz
<220>
   <223> Die Beschreibung von Künstliche Sequenz:Peptid
<400> 35
   Ile Ile Ile Gln Tyr Val Asp Asp Ile Leu
   1 5 10
<210> 36
   <211> 10
   <212> PRT
   <213> Künstliche Sequenz
<220>
   <223> Die Beschreibung von Künstliche Sequenz:Peptid
<400> 36
   Ile Ile Ile Gln Tyr Ile Asp Asp Ile Leu
   1 5 10
<210> 37
   <211> 10
   <212> PRT
   <213> Künstliche Sequenz
<220>
   <223> Die Beschreibung von Künstliche Sequenz:Peptid
<400> 37
   Ile Leu Ile Gln Tyr Val Asp Asp Ile Leu
   1 5 10
<210> 38
   <211> 10
   <212> PRT
   <213> Künstliche Sequenz
<220>
   <223> Die Beschreibung von Künstliche Sequenz:Peptid
<400> 38
   Ile Leu Ile Gln Tyr Ile Asp Asp Ile Leu
   1 5 10
<210> 39
   <211> 9
   <212> PRT
   <213> Künstliche Sequenz
<220>
   <223> Die Beschreibung von Künstliche Sequenz:Peptid
<400> 39
   Val Leu Tyr Gln Tyr Val Asp Asp Leu
   1 5
<210> 40
   <211> 9
   <212> PRT
   <213> Künstliche Sequenz
<220>
   <223> Die Beschreibung von Künstliche Sequenz:Peptid
<400> 40
   Val Leu Cys Gln Tyr Val Asp Asp Leu
   1 5
<210> 41
   <211> 9
   <212> PRT
   <213> Künstliche Sequenz
<220>
   <223> Die Beschreibung von Künstliche Sequenz:Peptid
<400> 41
   Val Leu Tyr Gln Tyr Ile Asp Asp Leu
   1 5
<210> 42
   <211> 9
   <212> PRT
   <213> Künstliche Sequenz
<220>
   <223> Die Beschreibung von Künstliche Sequenz:Peptid
<400> 42
   Val Leu Cys Gln Tyr Ile Asp Asp Leu
   1 5

## Beschreibung

Die Erfindung betrifft eine Verbindung bzw. ein Arzneimittel zur Induktion von zytotoxischen T-Zellen. Die Erfindung betrifft ferner eine Verwendung des Arzneimittels zur Immunisierung gegen Retroviren, wie HIV-1, HIV-2, HTLV-I, HTLV-II, sowie gegen Viren wie Hepatitis-B.

Nach dem Stand der Technik ist es bekannt, daß durch Verabreichen bestimmter Arzneimittel zytotoxische T-Zellen (CTL) induziert werden können. Zytotoxische T-Zellen eliminieren u.a. spezifisch viral infizierte Zellen.

Zur Therapie der HIV-Infektion werden Hemmstoffe des viralen Enzyms Reverse Transcriptase eingesetzt. Ein wichtiger in der Klinik verwendeter Hemmer der Reversen Transcriptase ist das Medikament 3TC (=(-)2'-deoxy-3'-thiacytidine = Lamivudine). HIV kann jedoch gegen dieses Medikament resistent werden, in dem es am Kodon 194 der Reversen Transcriptase des Methionin zu einem Isoleucin bzw. Valin mutiert (PNAS, 1993: 90: 5653-6). Die gleiche Mutation bewirkt darüber hinaus auch eine Resistenz gegenüber anderen Reverse Transcriptase-Hemmern, wie 1592U89 (=Abacavir), Zalcitabin (DDC), Didanosin (DDI) und 2'Deoxy-5-Fluoro-3'thiacytidin (FTC). Auch andere Viren besitzen eine Reverse Transcriptase bzw. ähnliche DNS-Polymerasen, die wie die Reverse Transcriptase von HIV im aktiven katalytischen Zentrum die Sequenz YMDD enthalten. Die gleiche M zu V - Mutation in der YMDD-Sequenz der DNS-Polymerase des Hepatitis-B-Virus bewirkt auch beim Hepatitis-B-Virus eine Resistenz gegenüber dem gegen das Hepatitis-B-Virus aktive Medikament 3TC.

Aus der nachveröffentlichten WO 98/23755 sind zur Behandlung der multiplen Sklerose die folgenden Aminosäuresequenzen bekannt: TLVLQYVDDLLL und ILVLQYVDDLLL, wobei T = Threonin, V = Valin, I = Isoleucin, L = Leucin und Q = Glutamin bedeutet.

Der Artikel von N. Tanigaki et coll. (Immunogenetics (1994), 40:3, S. 192-198) offenbart die Bindung von 5 HLA-B27-Subtypen an Peptide, die eine Vielzahl von Ankerrestmotiven tragen, und insbesondere an Peptide, die Reste R an den Positionen 2 und 9 aufweisen.

Die Publikation von E. Harrer et coll. (J. INFECT. DIS. (1996), 173:2, S. 476-479) offenbart die Identifikation eines das YMDD-Motiv enthaltenden CTL-Epitops mit HLA-A2-Restriktion, das bei Retroviren sehr häufig erhalten und für die Funktion der RNAabhängigen DNA-Polymerase essentiell ist.

Aufgabe der Erfindung ist es, ein Arzneimittel anzugeben, mit dem Virusinfektionen, insbesondere HIV- oder Hepatitis B-Infektionen, wirksam blockiert bzw. in ihrem verlauf günstig beeinflußt werden können. Das Arzneimittel soll sowohl zur Verhinderung einer Infektion, z.B. in Form eines präventiven Vaccins, als auch zur Therapie einer etablierten Infektion geeignet sein.

Diese Aufgabe wird durch die Merkmale des Anspruchs 1 gelöst. Zweckmäßige Ausgestaltungen der Erfindung ergeben sich aus den Merkmalen der Ansprüche 2 bis 29.

Nach Maßgabe der Erfindung ist eine Verbindung bzw. ein Arzneimittel zur Induktion zytotoxischer T-Zellen vorgesehen, enthaltend oder bestehend aus einer Aminosäure oder einer diese Aminosäure kodierenden Nukleinsäure, wobei die Aminosäure die folgende Sequenz hat:
X1-Y-X2-D-D-X3,
wobei
- X1 =: mindestens eine beliebige Aminosäure,
- Y =: Tyrosin,
- X2 =: eine aus der folgenden Gruppe ausgewählte Aminosäure: Valin, Isoleucin, Leucin,
- D =: Aspartat, und
- X3 =: mindestens eine weitere beliebige Aminosäure,
wobei die folgenden Aminosäuresequenzen ausgenommen sind:
TLVLQYVDDLLL und ILVLQYVDDLLL, wobei T = Threonin, V = Valin, I = Isoleucin, L = Leucin und Q = Glutamin bedeutet, sowie ein Verfahren zur Verhinderung oder Behandlung einer Infektion mit Viren, bevorzugt mutierten HIV, HIV-1, HIV-2, HTLV-I, HTLV-II Viren oder mutierten Hepatitis-B-Viren oder einer Krankheit, die auf die Induktion von zytotoxischen T-Zellen anspricht, bestehend aus der Verabreichung einer wirksamen Menge eines Medikaments, umfassend eine Aminosäure, die die folgende Sequenz hat:
X1-Y-X2-D-D-X3, wobei
X1 = mindestens eine beliebige Aminosäure,
Y = Tyrosin,
X2 = eine aus der folgenden Gruppe ausgewählte Aminosäure: Valin (V), Isoleucin (I), Leucin (L),
D = Aspartat, und
X3 = mindestens eine weitere beliebige Aminosäure,
oder einer Nukleinsäure, die diese Aminosäue kodiert, an einen Patienten, bzw.
eine Verwendung der folgenden Aminosäure:
X1-Y-X2-D-D-X3, wobei
X1 = mindestens eine beliebige Aminosäure,
Y = Tyrosin,
X2 = eine aus der folgenden Gruppe ausgewählte Aminosäure: Valin (V), Isoleucin (I), Leucin (L),
D = Aspartat, und
X3 = mindestens eine weitere beliebige Aminosäure,
oder einer Nukleinsäure, die diese Aminosäuesequenz kodiert, zur Herstellung eines Medikaments zur Verhinderung oder Behandlung einer Infektion mit Viren, bevorzugt mutierten HIV, HIV-1, HIV-2, HTLV-I, HTLV-II Viren oder mutierten Hepatitis-B-Viren, oder einer Krankheit, die auf die Induktion von zytotoxischen T-Zellen anspricht.

Das erfindungsgemäße Arzneimittel induziert zytotoxische T-Zellen, welche insbesondere mit mutanten HIV-Viren infizierte Zellen zerstören. Die erfindungsgemäßen Sequenzen bilden überraschenderweise T-Zell-Epitope, die z.B. an das HLA-A2-Molekül binden und spezifische T-Zellrezeptoren gegen sich induzieren können. Damit gelingt es insbesondere, gezielt die bei der Behandlung mit dem Medikament 3TC und Abacavir auftretenden HIV-Mutanten zu bekämpfen.

Nach einem Ausgestaltungsmerkmal besteht die Aminosäuresequenz aus 9 Aminosäuren. X1 kann eine aus 4 oder 5, X3 eine aus einer oder 2 weiteren beliebigen Aminosäuren bestehende Sequenz sein. Eine solche Aminosäuresequenz eignet sich insbesondere zur Immunisierung gegen mutante HIV-Viren, aber auch gegen andere Viren, z.B. mutante Hepatitis B-Viren.

Zur Immunisierung kann die Aminosäuresequenz Bestandteil eines Peptids oder Proteins sein. Das Peptid oder Protein kann an ein Lipopeptid oder Lipoprotein, vorzugsweise an Tripalmitoyl-S-glycerylcysteinyl-seryl-serine, gekoppelt sein. Zweckmäßigerweise kann das Peptid oder Protein in einem Liposom oder ISCOM (=Immunostimulatory complex) enthalten sein. Das Peptid oder Protein kann aber auch an ein virales Protein gekoppelt sein, welches vorzugsweise aus der folgenden Gruppe ausgewählt ist: HIV-Virus-ähnliche-Partikel (=HIV-Virus-like Particles), HIV-Gag-Partikel oder HBs-Antigen.

Das Peptid kann vorzugsweise als Peptid-HLA-Komplex in löslicher Form, z.B. als HLA-A2-Tetramer, vorliegen. Der vorgenannte Komplex kann an ein Liposom gebunden sein. Das Peptid kann aber auch Bestandteil einer Antigen präsentierenden Zelle, vorzugsweise einer dendritischen Zelle, Makrophage, B-Zelle oder CD4⁺ T-Zelle, sein. Dies kann erreicht werden sowohl durch die exogene Zugabe des Peptides auf die Zelle als auch durch endogene Prozessierung von in den Zellen exprimierten Proteinen.

Das erfindungsgemäße Arzneimittel kann ferner Zytokine, wie Interleukin-2 und/oder GM-CSF, oder polyvalente Vakzine enthalten. Als besonders vorteilhaft hat es sich erwiesen, die Aminosäuresequenz aus einer der folgenden Sequenzen auszuwählen:
IVIYQYVDDL (SEQ ID NO:1), IVICQYVDDL (SEQ ID NO:2),
IVIYQYIDDL (SEQ ID NO:3), IVICQYIDDL (SEQ ID NO:4),
ITIYQYVDDL (SEQ ID NO:5), ITICQYVDDL (SEQ ID NO:6),
ITIYQYIDDL (SEQ ID NO:7), ITICQYIDDL (SEQ ID NO:8),
IIIYQYVDDL(SEQ ID NO:9), IIICQYVDDL (SEQ ID NO:10),
IIIYQYIDDL (SEQ ID NO:11), IIICQYIDDL (SEQ ID NO:12),
MVIYQYVDDL (SEQ ID NO:13), MVICQYVDDL (SEQ ID NO:14),
MVIYQYIDDL (SEQ ID NO:15), MVICQYIDDL (SEQ ID NO:16),
VIYQYVDDL (SEQ ID NO:17), VICQYVDDL (SEQ ID NO:18),
VIYQYIDDL (SEQ ID NO:19), VICQYIDDL (SEQ ID NO:20),
LIYQYVDDL (SEQ ID NO:21), LICQYVDDL (SEQ ID NO:22),
LIYQYIDDL (SEQ ID NO:23), LICQYIDDL (SEQ ID NO:24),
TILQYVDDILL (SEQ ID NO:25), TILQYIDDILL (SEQ ID NO:26),
ILQYVDDIL (SEQ ID NO:27), ILQYIDDIL (SEQ ID NO:28),
TIVQYVDDILL (SEQ ID NO:29), TIVQYIDDILL (SEQ ID NO:30),
IVQYIDDIL (SEQ ID NO:31), IVQYIDDIL (SEQ ID NO:32),
ILVQYVDDIL (SEQ ID NO:33), ILVQYIDDIL (SEQ ID NO:34),
IIIQYVDDIL (SEQ ID NO:35), IIIQYIDDIL (SEQ ID NO:36),
ILIQYVDDIL (SEQ ID NO:37), ILIQYIDDIL (SEQ ID NO:38),
VLYQYVDDL (SEQ ID NO:39), VLCQYVDDL (SEQ ID NO:40),
VLYQYIDDL(SEQ ID NO:41), VLCQYIDDL(SEQ ID NO:42),
wobei V = Valin, I = Isoleucin, L = Leucin, M = Methionin, C = Cystein und Q = Glutamin. Die Nukleinsäuresequenz kann eine

DNS oder RNS Nukleinsäuresequenz sein. Es ist möglich, daß die Nukleinsäuresequenz Bestandteil eines Plasmids oder eines viralen Vektors, vorzugsweise eines rekombinanten Vakzinia-Virus oder eines rekombinanten Adenovirus oder eines retroviralen Vektors ist. Die Nukleinsäuresequenz kann gleichfalls Bestandteil retroviraler Vektoren oder attenuierter Retroviren sein. Ferner kann die Nukleinsäure Bestandteil eines bakteriellen Vektors, vorzugsweise eines rekombinanten BCG- oder Salmonella-Vektors oder eines inaktivierten Virus- vorzugsweise eines HIV-Virus, sein. Das erfindungsgemäße Arzneimittel kann des weiteren zur Herstellung ex vivo von T-Zellen oder T-Zellenrezeptoren dienen.

Nach einer weiteren erfindungsgemäßen Lösung ist auch die Verwendung des erfindungsgemäßen Arzneimittels zur Prävention oder Behandlung einer Infektion mit Viren, vorzugsweise mutierter HIV-, HIV-1, HIV-2, HTLV-I, HTLV-II Viren oder mutierter Hepatitis B-Viren. Die Viren können mutante Viren mit einer Resistenz gegen (-)-2',3'-Dideoxy-3'-thiacytidin [=3TC (Lamivudine)], (-)-(1S,4R)-4-[2-amino-6-(cyclopropylamino)-9H-purin-9-yl]-2-cyclopenten-1-methanol [=Abacavir], 2',3'-Dideoxyinosin [=Didanosin], 2',3'-Didesoxycytidin [=Zalcitabin], (-)-2'-deoxy-5-fluoro-3'-thiacytidin [=FTC] sein.

Die Wirksamkeit des erfindungsgemäßen Arzneimittels wird beispielhaft anhand graphisch dargestellter Versuchsergebnisse erläutert. Hierin zeigen:
- Fig. 1: die Erkennung des CTL Epitops VIYQYVDDL(SEQ ID NO:17) bzw. VIYQYIDDL(SEQ ID NO:19) durch den CTL Klon ETMV1 und die fehlende Erkennung eines früher beschriebenen CTL Epitops VIYQYMDDL durch den gleichen Klon,
- Fig. 2: die spezifische Lyse des Klons des ETMV1 bei Titrierung der Peptide VIYQYVDDL (SEQ ID NO:17) und VIYQYIDDL (SEQ ID NO:19)
- Fig. 3: die Erkennung der Peptide VIYQYVDDL(SEQ ID NO:17) und
- Fig. 4: die fehlende Erkennung der Peptide VIYQYVDDL(SEQ ID NO:17) und VIYQYIDDL (SEQ ID NO:19) durch den CTL Klon EB3, welcher die Wildtypsequenz VIYQYMDDL erkennt.

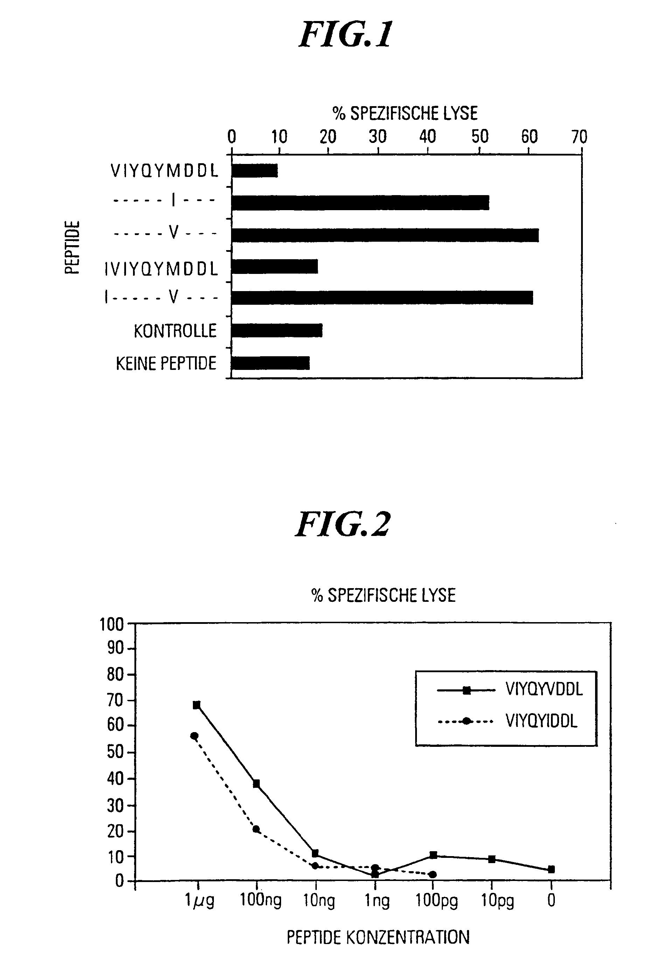

Die in Fig. 1 ausgewiesenen Peptide wurden mit einer Konzentration von 1 µg/ml mit ⁵¹Chrom markierten autologen EBV-transformierten B-Zell-Linien 1 Stunde vorinkubiert. Vier Stunden nach Zugabe des Klons ETMV1 mit einem Effektor-Target-Verhältnis von 15:1 wurden die Überstände geerntet und die spezifische Lyse anhand der Chromfreisetzung berechnet. Bei der Kontrolle wurde das p17-Peptid KIRLRPGGK verwendet. Wie aus Fig. 1 ersichtlich ist, sind nur die Peptide IVIYQYVDDL (SEQ ID NO:1), VIYQYVDDL (SEQ ID NO:17), VIYQYIDDL (SEQ ID NO:19) erkannt worden, welche die Restistenzmutationen gegen Reverse Transpcriptase Hemmer tragen. Das Wildtyppeptid VIYQYMDDL ist nicht erkannt worden.

Zur Erlangung der in Fig. 2 dargestellten Ergebnisse wurden die darin ausgewiesenen Peptide mit den angegebenen Konzentrationen mit ⁵¹Chrom markierten autologen EBV-transformierten B-Zell-Linien 1 Stunde vorinkubiert. 4 Stunden nach Zugabe des Klons ETMV1 mit einem Effektor-Target-Verhältnis von 5:1 wurden die Überstände geerntet und die spezifische Lyse anhand der Chromfreisetzung berechnet.

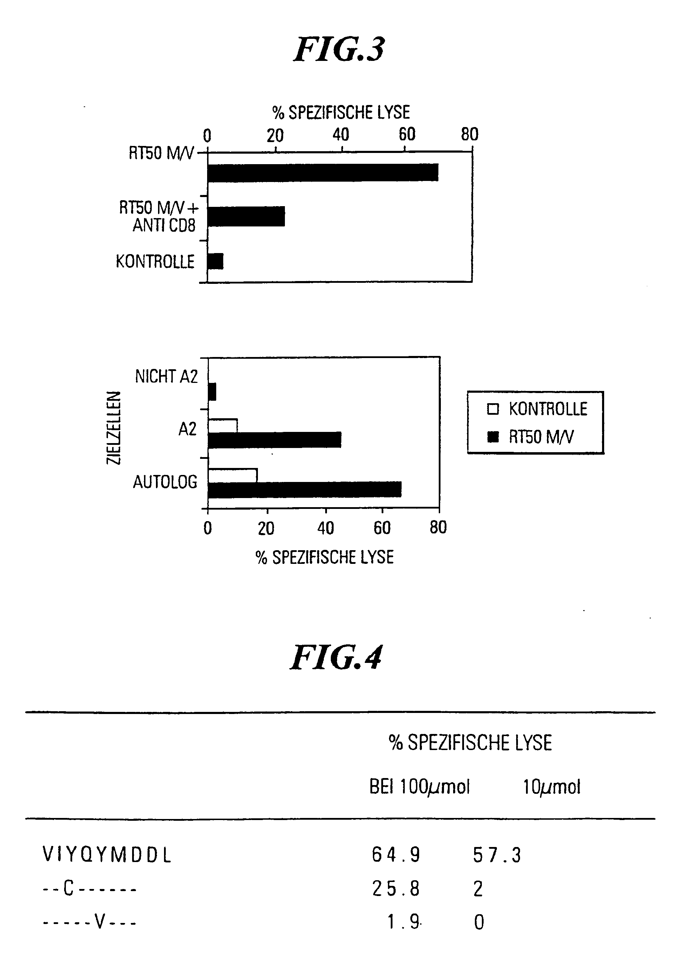

Fig. 3 zeigt die Erkennung des Peptids VIYQYVDDL (SEQ ID NO:17) (=RT50 M/V). Sie ist HLA-A2 restringiert. Die gezeigten Ergebnisse wurden dadurch erzielt, daß das Peptid bzw. ein Kontrollpeptid in einer Konzentration von 10µg/ml mit ⁵¹ Chrom markierten autologen EBV-transformierten B-Zell-Linien bzw. HLA-A2-gematchten bzw. HLA-A2-negativen allogenen B-Zell-Linien eine Stunde vorinkubiert wurden. 4 Stunden nach Zugabe des Klons ETMV1 mit einem Effektor-Target-Verhältnis von 5:1 wurden die Überstände geerntet und die spezifische Lyse anhand der Chromfreisetzung berechnet. Die Zugabe von Antikörpern gegen CD8 zeigt, daß die Lyse HLA Klasse-I restringiert ist.

Aus der in Fig. 4 gezeigten Tabelle ist die Erkennung von Variantenpeptiden durch Klon EB3 ersichtlich. Dazu wurden Peptide mit den angegebenen Konzentrationen mit ⁵¹ Chrom markierten autologen EBV-transformierten B-Zell-Linien für eine Stunde vorinkubiert. 5 Stunden nach Zugabe des Klons EB3 mit einem Effektor-Target-Verhältnis von 8:1 bzw. 10:1 wurden die Überstände geerntet und die spezifische Lyse anhand der Chromfreisetzung berechnet. Dieser Klon erkennt die unmutierte Wildtypsequenz von HIV, aber nicht die erfindungsgemäße Sequenz. Das zeigt, daß es sich bei der erfindungsgemäßen Sequenz um ein neues CTL Epitop handelt.

## Patentansprüche

1. Verbindungsequenz enthaltend oder bestehend aus einer Aminosäuresequenz oder einer diese Aminosäuresequenz kodierenden Nukleinsäure, wobei die Aminosäuresequenz die folgende Sequenz hat:
X1-Y-X2-D-D-X3, wobei
X1= mindestens eine beliebige Aminosäure,
Y= Tyrosin,
X2= eine aus der folgenden Gruppe ausgewählte Aminosäure: Valin, Isoleucin, Leucin,
D= Aspartat, und
X3= mindestens eine weitere beliebige Aminosäure, wobei die folgenden Aminosäuresequenzen ausgenommen sind: LRVEYLDDR, TLVLQYVDDLLL und ILVLQYVDDLLL, wobei T= Threonin, V= Valin, I= Isoleucin, L= Leucin, Q= Glutamin und R= Arginin bedeutet.

2. Verbindung nach Anspruch 1, wobei die Aminosäuresequenz aus 9 Aminosäuren besteht.

3. Verbindung nach einem der vorhergehenden Ansprüche, wobei X1 eine aus 4 oder 5 Aminosäuren bestehende Sequenz ist.

4. Verbindung nach einem der vorhergehenden Ansprüche, wobei X3 eine aus einer oder 2 weiteren Aminosäuren bestehende Sequenz ist.

5. Verbindung nach einem der vorhergehenden Ansprüche, wobei die Aminosäuresequenz Bestandteil eines Peptids ist.

6. Verbindung nach einem der vorhergehenden Ansprüche, wobei die Aminosäuresequenz Bestandteil eines Proteins ist.

7. Verbindung nach einem der vorhergehenden Ansprüche, wobei das Peptid oder Protein an ein Lipopeptid oder Lipoprotein, vorzugsweise an Tripalmitoyl-S-glycerylcysteinyl-seryl-serine, gekoppelt ist.

8. Verbindung nach einem der vorhergehenden Ansprüche, wobei das Peptid oder Protein in einem Liposom oder ISCOM enthalten ist.

9. Verbindung nach einem der vorhergehenden Ansprüche, wobei das Peptid oder Protein an ein virales Protein gekoppelt ist.

10. Verbindung nach einem der vorhergehenden Ansprüche, wobei das virale Protein aus der folgenden Gruppe ausgewählt ist: HIV-virus-ähnliche-Partikel (=HIV-Virus-like particles), HIV-Gag-Partikel oder HBs-Antigen.

11. Verbindung nach einem der vorhergehenden Ansprüche, wobei das Peptid als Peptid-HLA-Komplex in löslicher Form, vorzugsweise als HLA-A2-Tetramer, vorliegt.

12. Verbindung nach einem der vorhergehenden Ansprüche, wobei der Peptid-HLA-Komplex an ein Liposom gebunden ist.

13. Verbindung nach einem der vorhergehenden Ansprüche, wobei das Peptid Bestandteil einer antigenprasentierenden Zelle, vorzugsweise einer dendritischen, Zelle, Makrophage, B-Zelle oder CD4⁺ T-zelle, ist.

14. Verbindung nach einem der vorhergehenden Ansprüche, wobei die Aminosäuresequnz aus einer der folgenden Sequenzen, ausgewählt ist:
IVIYQYVDDL (SEQ ID NO:1), IVICQYVDDL (SEQ ID NO:2),
IVIYQYIDDL (SEQ ID NO:3), IVICQYIDDL (SEQ ID NO:4),
ITIYQYVDDL (SEQ ID NO:5), ITICQYVDDL (SEQ ID. NO*:* 6),
ITIYQYIDDL (SEQ ID NO:7), ITICQYIDDL (SEQ ID NO:8),
IIIYQYVDDL (SEQ ID NO:9), IIICQYVDDL (SEQ ID NO:10),
IIIYQYIDDL(SEQ ID NO:11), IIICQYIDDL (SEQ ID NO:12),
MVYYQYVDDL(SEQ ID NO*:*13), MVICQYVDDL (SEQ ID NO:14),
MVIYQYIDDL(SEQ ID NO:15), MVICQYIDDL (SEQ ID NO:16),
VIYQYVDDL (SEQ ID NO:17), VICQYVDDL (SEQ ID NO:18),
VIYQYIDDL (SEQ ID NO:19), VICQYIDDL (SEQ ID NO:20),
LIYQYVDDL (SEQ ID NO:21), LICQYVDDL (SEQ ID NO:22),
LIYQYIDDL (SEQ ID NO:23), LICQYIDDL (SEQ ID NO:24),
TILQYVDDILL (SEQ ID NO:25), TILQYIDDILL(SEQ ID NO:26),
ILQYVDDIL (SEQ ID NO:27), ILQYIDDIL (SEQ ID NO:28),
TIVQYVDDILL (SEQ ID NO:29), TIVQYIDDILL (SEQ ID NO:30),
IVQYIDDIL (SEQ ID NO:31), IVQYIDDIL (SEQ ID NO:32),
ILVQYVDDIL(SEQ ID NO:33), ILVQYIDDIL (SEQ ID NO:34),
IIIQYVDDIL(SEQ ID NO:35), IIIQYIDDIL (SEQ ID NO:36),
ILIQYVDDIL (SEQ ID NO:37), ILIQYIDDIL (SEQ ID NO:38),
VLYQYVDDL(SEQ ID NO:39), VLCQYVDDL (SEQ ID NO:40),
VLYQYIDDL(SEQ ID NO:41), VLCQYIDDL(SEQ ID NO:42),
wobei V = Valin, I = Isoleucin, L = Leucin, M = Methionin, C = Cystein und Q = Glutamin.

15. Verbindung nach einem der Ansprüche 1,2, 3, 4, 13 oder 14, wobei die Nukleinsauresequenz eine DNS oder RNS-Nukleinsäuresequenz ist.

16. Verbindung nach einem der vorhergehenden Ansprüche, wobei die Nukleinsäuresequenz Bestandteil eines Plasmids oder eines viralen Vektors, vorzugsweise eines rekombinanten Vaccinia-Virus oder Adenovirus oder eines retroviralen Vektors ist.

17. Verbindung nach einem der vorhergehenden Ansprüche, wobei die Nukleinsäuresequenz Bestandteil eines bakteriellen Vektors, vorzugsweise eines rekombinanten BCG- oder Salmonella-Vektors, ist.

18. Verbindung nach einem der vorhergehenden Ansprüche, wobei die Nukleinsäuresequenz Bestandteil eines inaktivierten Virus, vorzugsweise eines HIV-Virus, ist.

19. Arzneimittel enthaltend als Wirkstoff eine Verbindung nach einem der vorhergehenden Ansprüche.

20. Arzneimittel gemäss Anspruch 19 in Form eines Impfstoffs.

21. Arzneimittel gemäss Anspruch 20, wobei polyvalente Vakzine enthalten sind.

22. Arzneimittel gemäss Ansprüche 19-21, wobei ein oder mehrere Zytokine als Adjuvans enthalten sind.

23. Arzneimittel gemäss Anspruch 19-22, wobei als Zytokin Interleukin-2 und/oder GM-CSF enthalten ist.

24. Verwendung der folgenden Aminosäuresequenz:
X1-Y-X2-D-D-X3, wobei
X1 = mindestens eine beliebige Aminosäure,
Y = Tyrosin,
X2 = eine aus der folgenden Gruppe ausgewählte Aminosäure: Valin (V), Isoleucin (I), Leucin (L),
D = Aspartat, und
X3 = mindestens eine weitere beliebige Aminosäure, oder einer Nukleinsäure, die diese Aminosäuesequenz kodiert, zur Herstellung eines Medikaments zur Verhinderung oder Behandlung einer Infektion mit mutierten HIV, HIV-1, HIV-2, HTLV-I, HTLV-II Viren oder mutierten Hepatitis-B-Viren.

25. Verwendung nach Anspruch 24, wobei die Viren mutante Viren mit einer Resistenz gegen Reverse Transcriptase Hemmer sind.

26. Verwendung nach Anspruch 24 oder 25, wobei die Viren mutante Viren mit einer Resistenz gegen (-)-2',3'-Dideoxy-3'-thiacytidin [-3TC (Lamivudine)], (-)-(1S,4R)-4-[2-amino-6-(cyclopropylamino)-9H-purin-9-yl]-2-cyclo pentene-1-methanol [=Abacavir], 2', 3'-Dideoxyinosin [=Didanosin], 2', 3'-Didesoxycytidin [=Zalcitabin], (-)-2'-deoxy-5-fluoro-3'-thiacytidin [=FTC] sind.

## Claims

1. Compound comprising or consisting of an amino acid sequence or a nucleic acid coding for this amino acid sequence which has the following sequence:
X1-Y-X2-D-D-X3, where
X1= at least one random amino acid,
Y= tyrosine,
X2= an amino acid selected from the following group: valine, isoleucine, leucine,
D= aspartate, and
X3= at least one further random amino acid, except the following amino acid sequences: LRVEYLDDR, TLVLQYVDDLLL and ILVLQYVDDLLL, where T= threonine, V= valine, I= isoleucine, L= leucine, Q= glutamine and R= arginine.

2. Compound according to Claim 1, wherein the amino acid sequence consists of 9 amino acids.

3. Compound according to either of the preceding claims, wherein X1 is a sequence consisting of 4 or 5 amino acids.

4. Compound according to any of the preceding claims, wherein X3 is a sequence consisting of one or 2 further amino acids.

5. Compound according to any of the preceding claims, wherein the amino acid sequence is part of a peptide.

6. Compound according to any of the preceding claims, wherein the amino acid sequence is part of a protein.

7. Compound according to any of the preceding claims, wherein the peptide or protein is coupled to a lipopeptide or lipoprotein, preferably to tripalmitoyl-S-glycerylcysteinyl-seryl-serine.

8. Compound according to any of the preceding claims, wherein the peptide or protein is present in a liposome or ISCOM.

9. Compound according to any of the preceding claims, wherein the peptide or protein is coupled to a viral protein.

10. Compound according to any of the preceding claims, wherein the viral protein is selected from the following group: HIV virus-like particles, HIV gag particles or HBs antigen.

11. Compound according to any of the preceding claims, wherein the peptide is present as peptide-HLA complex in soluble form, preferably as HLA A2 tetramer.

12. Compound according to any of the preceding claims, wherein the peptide-HLA complex is bound to a liposome.

13. Compound according to any of the preceding claims, wherein the peptide is part of an antigen-presenting cell, preferably of a dendritic cell, macrophage, B cell or CD4⁺ T cell.

14. Compound according to any of the preceding claims, wherein the amino acid sequence is selected from any of the following sequences:
IVIYQYVDDL (SEQ ID NO:1), IVICQYVDDL (SEQ ID NO:2),
IVIYQYIDDL (SEQ ID NO:3), IVICQYIDDL (SEQ ID NO:4),
ITIYQYVDDL (SEQ ID NO:5), ITICQYVDDL (SEQ ID NO:6),
ITIYQYIDDL (SEQ ID NO:7), ITICQYIDDL (SEQ ID NO:8),
IIIYQYVDDL (SEQ ID NO:9), IIICQYVDDL (SEQ ID NO:10),
IIIYQYIDDL (SEQ ID NO:11), IIICQYIDDL (SEQ ID NO:12),
MVIYQYVDDL (SEQ ID NO:13), MVICQYVDDL (SEQ ID NO:14),
MVIYQYIDDL (SEQ ID NO:15), MVICQYIDDL (SEQ ID NO:16),
VIYQYVDDL (SEQ ID NO:17), VICQYVDDL (SEQ ID NO:18),
VIYQYIDDL (SEQ ID NO:19), VICQYIDDL (SEQ ID NO:20),
LIYQYVDDL (SEQ ID NO:21), LICQYVDDL (SEQ ID NO:22),
LIYQYIDDL (SEQ ID NO:23), LICQYIDDL (SEQ ID NO:24),
TILQYVDDILL (SEQ ID NO:25), TILQYIDDILL (SEQ ID NO:26),
ILQYVDDIL (SEQ ID NO:27), ILQYIDDIL (SEQ ID NO:28),
TIVQYVDDILL (SEQ ID NO:29), TIVQYIDDILL (SEQ ID NO:30),
IVQYIDDIL (SEQ ID NO:31), IVQYIDDIL (SEQ ID NO:32),
ILVQYVDDIL (SEQ ID NO:33), ILVQYIDDIL (SEQ ID NO:34),
IIIQYVDDIL (SEQ ID NO:35), IIIQYIDDIL (SEQ ID NO:36),
ILIQYVDDIL (SEQ ID NO:37), ILIQYIDDIL (SEQ ID NO:38),
VLYQYVDDL (SEQ ID NO:39), VLCQYVDDL (SEQ ID NO:40),
VLYQYIDDL (SEQ ID NO:41), VLCQYIDDL (SEQ ID NO:42),
where V = valine, I = isoleucine, L = leucine, M = methionine, C = cysteine and Q = glutamine.

15. Compound according to any of Claims 1, 2, 3, 4, 13 or 14, wherein the nucleic acid sequence is a DNA or an RNA nucleic acid sequence.

16. Compound according to any of the preceding claims, wherein the nucleic acid sequence is part of a plasmid or of a viral vector, preferably of a recombinant vaccinia virus or adenovirus, or of a retroviral vector.

17. Compound according to any of the preceding claims, wherein the nucleic acid sequence is part of a bacterial vector, preferably of a recombinant BCG or Salmonella vector.

18. Compound according to any of the preceding claims, wherein the nucleic acid sequence is part of an inactivated virus, preferably of an HIV virus.

19. Medicament comprising a compound according to any of the preceding claims as active substance.

20. Medicament according to Claim 19 in the form of a vaccine.

21. Medicament according to Claim 20, which comprises polyvalent vaccines.

22. Medicament according to Claims 19-21, which comprises one or more cytokines as adjuvants.

23. Medicament according to Claims 19-22, which comprises the cytokine interleukin-2 and/or GM-CSF as cytokine.

24. Use of the following amino acid sequence:
X1-Y-X2-D-D-X3, where
X1= at least one random amino acid,
Y= tyrosine,
X2= an amino acid selected from the following group: valine (V), isoleucine (I), leucine (L),
D= aspartate, and
X3= at least one further random amino acid,
or of a nucleic acid coding for this amino acid sequence for preparing a drug for preventing or treating an infection with mutated HIV, HIV 1, HIV 2, HTLV I, HTLV II viruses or mutated hepatitis B viruses.

25. Use according to Claim 24, wherein the viruses are mutant viruses having a resistance to reverse transcriptase inhibitors.

26. Use according to Claim 24 or 25, wherein the viruses are mutant viruses having a resistance to (-)-2',3'-dideoxy-3'-thiacytidine [= 3TC (lamivudine)], (-)-(1S,4R)-4-[2-amino-6-(cyclopropylamino)-9H-purin-9-yl]-2-cyclopentene-1-methanol [= abacavir], 2',3'-dideoxyinosine [= didanosin], 2',3'-dideoxycytidine [= zalcitabin], (-)-2'-deoxy-5-fluoro-3'-thiacytidine [=FTC].

## Revendications

1. Composé contenant ou constitué d'une séquence d'acides aminés ou d'un acide nucléique codant cette séquence d'acides aminés, la séquence d'acides aminés présentant la séquence suivante:
X1-Y-X2-D-D-X3, dans laquelle
X1 = au moins un acide aminé quelconque
Y = tyrosine
X2 = un acide aminé choisi dans le groupe suivant: valine, isoleucine, leucine D = aspartate, et
X3 = au moins un autre acide aminé quelconque, à l'exclusion des séquences d'acides aminés suivantes :
LRVEYLDDR, TLVLQYVDDLLL et ILVLQYVDDLLL, dans lesquelles T = thréonine, V = valine, I = isoleucine, L = leucine, Q = glutamine et R = arginine.

2. Composé selon la revendication 1 dans lequel la séquence d'acides aminés est constituée de 9 acides aminés.

3. Composé selon l'une des revendications précédentes dans lequel X1 est une séquence constituée de 4 ou 5 acides aminés.

4. Composé selon l'une des revendications précédentes, dans lequel X3 est une séquence constituée d'un ou de 2 autres acides aminés.

5. Composé selon l'une des revendications précédentes, dans lequel la séquence d'acides aminés est un élément d'un peptide.

6. Composé selon l'une des revendications précédentes dans lequel la séquence d'acides aminés est un élément d'une protéine.

7. Composé selon l'une des revendications précédentes, dans lequel le peptide ou la protéine est couplé(e) à un lipopeptide ou à une lipoprotéine, de préférence à la tripalmitoyl-S-glycérylcystéinyl-séryl-sérine.

8. Composé selon l'une des revendications précédentes, dans lequel le peptide ou la protéine est contenu dans un liposome ou un ISCOM.

9. Composé selon l'une des revendications précédentes, dans lequel le peptide ou la protéine est couplé à une protéine virale.

10. Composé selon l'une des revendications précédentes, dans lequel la protéine virale est choisie parmi le groupe suivant: particule de type virus-VIH, (HIV-virus-like particle), particules VIH-Gag ou antigène-HBs.

11. Composé selon l'une des revendications précédentes, dans laquelle le peptide se présente sous la forme d'un complexe peptide-HLA sous forme soluble, de préférence sous la forme d'un tétramère HLA-A2.

12. Composé selon l'une des revendications précédentes, dans lequel le complexe peptide-HLA est lié à un liposome.

13. Composé selon l'une des revendications précédentes, dans lequel le peptide est un composant d'une cellule présentatrice d'antigène, de préférence une cellule dendritique, un macrophage, une cellule B ou une cellule CD4+ T.

14. Composé selon l'une des revendications précédentes, dans lequel la séquence d'acides aminés est choisie parmi l'une des séquences suivantes:
IVIYQYVDDL(SEQ ID NO:1), IVICQYVDDL(SEQ ID NO:2),
IVIYQYIDDL(SEQ ID NO:3), IVICQYIDDL(SEQ ID NO:4),
ITIYQYVDDL(SEQ ID NO:5), ITICQYVDDL(SEQ ID NO:6),
ITIYQYIDDL (SEQ ID NO:7), ITICQYIDDL(SEQ ID NO:8),
IIIYQYVDDL (SEQ ID NO:9), IIICQYVDDL (SEQ ID NO:10),
IIIYQYIDDL (SEQ ID NO:11), IIICQYIDDL (SEQ ID NO:12),
MVIYQYVDDL (SEQ ID NO:13), MVICQYVDDL (SEQ ID NO:14),
MVIYQYIDDL (SEQ ID NO:15), MVICQYIDDL (SEQ ID NO:16),
VIYQYVDDL (SEQ ID NO:17), VICQYVDDL (SEQ ID NO:18),
VIYQYIDDL (SEQ ID NO:19), VICQYIDDL (SEQ ID NO:20),
LIYQYVDDL (SEQ ID NO:21), LICQYVDDL (SEQ ID NO:22),
LIYQYIDDL (SEQ ID NO:23), LICQYIDDL (SEQ ID NO:24),
TILQYVDDILL (SEQ ID NO:25), TILQYIDDILL (SEQ ID NO:26),
ILQYVDDIL (SEQ ID NO:27), ILQYIDDIL (SEQ ID NO:28),
TIVQYVDDILL (SEQ ID NO:29), TIVQYIDDILL (SEQ ID NO:30),
IVQYIDDIL(SEQ ID NO:31), IVQYIDDIL (SEQ ID NO:32),
ILVQYVDDIL(SEQ ID NO:33), ILVQYIDDIL (SEQ ID NO:34),
IIIQYVDDIL (SEQ ID NO:35), IIIQYIDDIL (SEQ ID NO:36),
ILIQYVDDIL(SEQ ID NO:37), ILIQYIDDIL (SEQ ID NO:38),
VLYQYVDDL (SEQ ID NO:39), VLCQYVDDL (SEQ ID NO:40),
VLYQYIDDL (SEQ ID NO:41), VLCQYIDDL (SEQ ID NO:42),
dans lesquelles V = valine, I = isoleucine, L = leucine, M = méthionine, C = cystéine et Q = glutamine.

15. Composé selon l'une des revendications 1, 2, 3, 4, 13 ou 14, dans lequel la séquence d'acide nucléique est une séquence d'acide nucléique ADN ou ARN.

16. Composé selon l'une des revendications précédentes, dans lequel la séquence d'acide nucléique est un élément d'un plasmide ou d'un vecteur viral, de préférence un virus de vaccine recombinant, ou un adénovirus recombinant ou d'un vecteur rétroviral.

17. Composé selon l'une des revendications précédentes, dans lequel la séquence d'acide nucléique est un élément d'un vecteur bactérien, de préférence d'un vecteur de BCG ou de salmonelle recombinant.

18. Composé selon l'une des revendications précédentes, dans lequel la séquence d'acide nucléique est un élément d'un virus inactivé, de préférence d'un virus VIH.

19. Médicament contenant comme ingrédient actif un composé selon l'une des revendications précédentes.

20. Médicament selon la revendication 19 sous la forme d'un vaccin.

21. Médicament selon la revendication 20, dans lequel des vaccins polyvalents sont incorporés.

22. Médicament selon les revendications 19 à 21, dans lequel une ou plusieurs cytokines sont incorporées comme adjuvant.

23. Médicament selon les revendications 19 à 22, dans lequel l'interleukine-2 et/ou le GM-CSF est incorporé comme cytokine.

24. Utilisation des séquences d'acides aminés suivantes:
X1-Y-X2-D-D-X3, dans lesquelles
X1 = au moins un acide aminé quelconque
Y = tyrosine
X2 = un acide aminé choisi parmi le groupe suivant: valine (V), isoleucine (I), leucine (L),
D = aspartate, et
X3 = au moins un autre acide aminé quelconque, ou un acide nucléique, qui code cette séquence d'acides aminés, pour la fabrication d'un médicament pour inhiber ou traiter une infection avec les virus VIH, VIH-1, VIH-2, HTLV-I, HTLV-II mutés ou les virus de l'hépatite-B mutés.

25. Utilisation selon la revendication 24, dans laquelle les virus sont des virus mutants comportant une résistance aux inhibiteurs de la transcriptase inverse.

26. Utilisation selon la revendication 24 ou 25, dans laquelle les virus sont des virus mutants comportant une résistance à (-)-2',3'-didésoxy-3'-thiacytidine [=3TC (Lamivudine)], (-)-(1S, 4R)-4-[2-amino-6-(cyclopropylamino)-9H-purine-9-yl]-2-cyclopentène-1-méthanol [=Abacavir], 2',3'-didésoxyinosine [-Didanosine], 2',3'-didésoxycytidine [=Zalcitabine], (-)-2'-désoxy-5-fluoro-3'-thiacytidine [=FTC].
